# EUROPEAN PATENT APPLICATION

(11) **EP 4 810 495 A1**
(43) Date of publication of application: **23.09.2026**
(21) Application number: 25382256.3
(22) Date of filing: 19.03.2025
(51) Int. Cl.: C07D 471/04, A61K 31/437, A61P 5/08, A61P 25/32, A61P 35/00

(54) **RELACORILANT CRYSTALLINE FORMS**

(71) Applicant: CURIA SPAIN, S.A.U., 47151 Boecillo, Valladolid (ES)
(72) Inventor: IGLESIAS RETUERTO, Jesús Miguel, 47151 Boecillo - Valladolid (ES); FERREIRO GIL, Juan José, 47151 Boecillo - Valladolid (ES)
(74) Representative: ABG Intellectual Property Law, S.L.

(57) **Abstract**

The present invention relates to crystal solid forms of relacorilant, described herein as crystalline form I and form II, methods for preparing the same, pharmaceutical compositions containing them and their use in medicine, particularly for the treatment and/or prevention of Cushing's syndrome, solid tumors or alcoholism.

## Description

### Field of the Invention

The invention belongs to the field of pharmaceuticals, and more particularly relates to crystalline forms of relacorilant.

### Background of the Invention

Relacorilant (developmental code name CORT-125134) is the international non-proprietary name (INN) or common name of ((R)-(1-(4-fluorophenyl)-6-((1-methyl-1H-pyrazol-4-yl)sulfonyl)-4,4a,5,6,7,8-hexahydro-1H-pyrazolo[3,4-g]isoquinolin-4a-yl)(4-(trifluoromethyl)pyridine-2-yl)methanone), having the structure shown below:

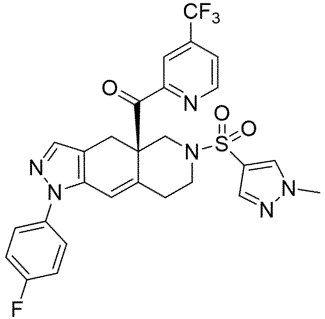

Relacorilant is an antiglucocorticoid which is under development for various indications including the treatment of Cushing's syndrome, solid tumors (such as ovarian, adrenal and prostate cancer) and alcoholism. The drug is a nonsteroidal compound which acts as a selective cortisol modulator that binds to the glucocorticoid receptor (GR), but does not bind to the body's other hormone receptors.

Relacorilant has been described previously in WO2013177559, example 18, where the crude product was isolated by chromatography on silica gel but no particular solid form was disclosed.

More recently, WO2022140600 described in example 8 the isolation of relacorilant as amorphous solid by precipitation.

In the formulation of drug compositions, it is important for the drug substance to be in a form in which it can be conveniently handled and processed. This is of importance, not only from the point of view of obtaining a commercially-viable manufacturing process, but also from the point of view of subsequent manufacture of pharmaceutical formulations comprising the active compound. Further, the active compound should exhibit a good performance in the pharmaceutical formulation in terms of chemical stability, bioavailability (absorption, dissolution rate...), etc.

Drug products are generally produced as solid materials. Even when formulated as liquids, the API is still usually produced as a solid prior to the final dissolution. Thus, the discovery of new solid forms of new or existing APIs such as relacorilant provides an opportunity to facilitate the manufacturing, handling and storage of the compound, and compositions containing it, and/or to improve the observed performance in the final dosage form.

Thus, there is a need for new solid forms of relacorilant having advantageous pharmaceutical properties such as, for example, one or more of processabiltiy, stability, and solubility.

### Summary of the Invention

The present invention solves the aforementioned need by the provision of crystal solid forms of relacorilant, described herein as crystalline forms I and II, which surprisingly have advantageous properties (e.g., improved stability and/or reproducibility). Up to date, to the best knowledge of the inventors, there is no crystalline form of relacorilant reported and therefore the crystalline forms of the invention represent a significant advancement in the development of appropriate pharmaceutical compositions of this API.

Thus, in a first aspect, the invention is directed to a crystalline form of relacorilant, designated herein as crystalline form I, characterized by an X-ray powder diffraction (XRPD) pattern comprising diffraction peaks at 2θ degrees of about 20.2±0.2°, 19.5±0.2° and 21.2±0.2°.

Additionally or alternatively, the crystalline form I of relacorilant is characterized by a thermogravimetric analysis (TGA) thermogram showing a weight loss of about 3.8 % upon heating up to about 120 °C.

Additionally or alternatively, the crystalline form I of relacorilant is characterized by a differential scanning calorimetry (DSC) thermogram having an endotherm with an onset of about 100-101 °C.

Additionally or alternatively, the crystalline form I of relacorilant is characterized by bands at about 1740, 1686, 1513, 1440, 1420 and 1285 cm⁻¹ in a fourier-transform infrared spectroscopy (FTIR) spectrum.

In a second aspect, the invention is directed to a crystalline form of relacorilant, designated herein as crystalline form II, characterized by an X-ray powder diffraction (XRPD) pattern comprising diffraction peaks at 2θ degrees of about 10.4±0.2°, 20.5±0.2°, 17.1±0.2° and 19.6±0.2°.

Additionally or alternatively, the crystalline form II of relacorilant is characterized by a thermogravimetric analysis (TGA) thermogram showing a weight loss of about 0.4 % upon heating up to about 75 °C.

Additionally or alternatively, the crystalline form II of relacorilant is characterized by a differential scanning calorimetry (DSC) thermogram having an endotherm with an onset of about 102-103 °C.

Additionally or alternatively, the crystalline form II of relacorilant is characterized by bands at about 1686, 1515, 1334 and 1164 cm⁻¹ in a fourier-transform infrared spectroscopy (FTIR) spectrum.

In a further aspect, the invention is directed to a method for preparing the crystalline form I of relacorilant, said method comprising using ethyl acetate as crystallization solvent and optionally adding heptane as anti-solvent until obtaining a suspension.

In a further aspect, the invention is directed to a method for preparing the crystalline form II of relacorilant, said method comprising using methanol as crystallization solvent.

In a further aspect, the invention is directed to a crystalline form of relacorilant obtainable by anyone of the above methods.

In a further aspect, the invention is directed to a pharmaceutical composition or a medicament comprising:
the crystalline form I and/or II of relacorilant, and
a pharmaceutically acceptable excipient.

In a further aspect, the invention is directed to the crystalline form I and/or II of relacorilant or a pharmaceutical composition thereof for use in medicine, particularly for use in the treatment and/or prevention of Cushing's syndrome, solid tumors or alcoholism.

Another aspect of this invention refers to the use of the crystalline form I and/or II of relacorilant in the manufacture of a medicament for the treatment and/or prevention of Cushing's syndrome, solid tumors or alcoholism.

Another aspect of the present invention refers to a method for the treatment and/or prevention of Cushing's syndrome, solid tumors or alcoholism in a subject, said method comprising the administration of a therapeutically effective amount of the crystalline form I and/or II of relacorilant or a pharmaceutical composition thereof to said subject. In a preferred embodiment, said subject is a human being.

These aspects and preferred embodiments thereof are additionally also defined hereinafter in the detailed description and in the claims.

All the features described in this specification (including the claims, description and drawings) can be combined in any combination, with the exception of combinations of such mutually exclusive features.

### Brief description of Drawings

To better understand the invention, its objects and advantages, the following figures are attached to the specification in which the following is depicted:
**Figure 1** shows an illustrative X-ray powder diffraction (XRPD) pattern of amorphous relacorilant prepared by the method of Example 1 (comparative).
**Figure 2** shows an illustrative thermogravimetric analysis (TGA) of the amorphous form of relacorilant prepared by the method of Example 1 (comparative).
**Figure 3** shows an illustrative differential scanning calorimetry (DSC) thermogram of the amorphous form of relacorilant prepared by the method of Example 1 (comparative).
**Figure 4** shows an illustrative fourier-transform infrared spectroscopy (FTIR) spectrum of the amorphous form of relacorilant prepared by the method of Example 1 (comparative).
**Figure 5** shows an illustrative X-ray powder diffraction (XRPD) pattern of the crystalline form I of relacorilant prepared by the method of Example 2.
**Figure 6** shows an illustrative thermogravimetric analysis (TGA) of the crystalline form I of relacorilant prepared by the method of Example 2.
**Figure 7** shows an illustrative differential scanning calorimetry (DSC) thermogram of the crystalline form I of relacorilant prepared by the method of Example 2.
**Figure 8** shows an illustrative fourier-transform infrared spectroscopy (FTIR) spectrum of the crystalline form I of relacorilant prepared by the method of Example 2.
**Figure 9** shows an illustrative X-ray powder diffraction (XRPD) pattern of the crystalline form II of relacorilant prepared by the method of Example 3.
**Figure 10** shows an illustrative thermogravimetric analysis (TGA) of the crystalline form II of relacorilant prepared by the method of Example 3.
**Figure 11** shows an illustrative differential scanning calorimetry (DSC) thermogram of the crystalline form II of relacorilant prepared by the method of Example 3.
**Figure 12** shows an illustrative fourier-transform infrared spectroscopy (FTIR) spectrum of the crystalline form II of relacorilant prepared by the method of Example 3.
**Figure 13** shows an illustrative X-ray powder diffraction (XRPD) pattern of amorphous form of relacorilant after 1 month under accelerated stability conditions.

### Detailed Description of the Invention

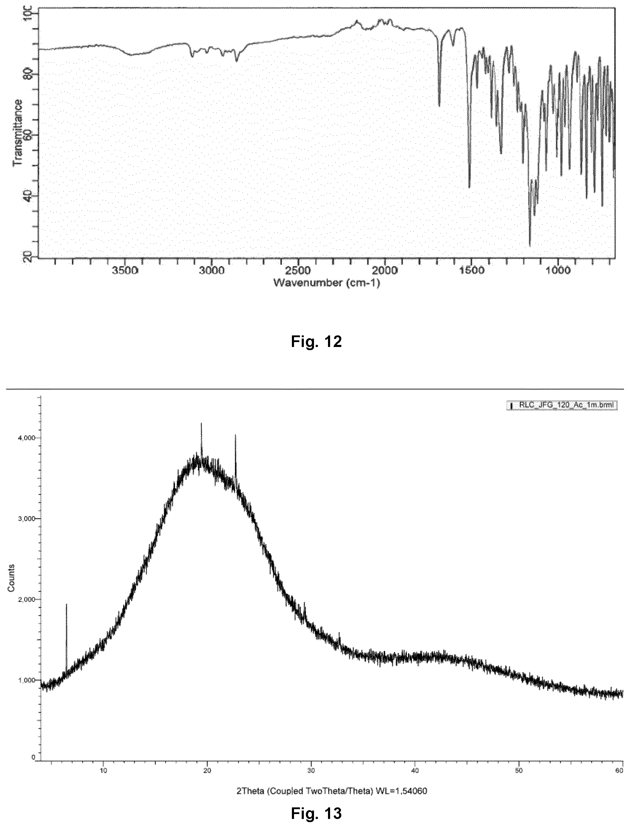

Relacorilant is disclosed in the prior art in amorphous form. It is found through research that the amorphous relacorilant has disadvantages such as poor chemical stability, hygroscopicity and easy degradability, which is not suitable for medicine use and industrial production. In particular, some impurity peaks are found in the XRPD pattern of the amorphous form of relacorilant at only 1 month of storage under conditions of accelerated testing (40 °C ± 2 °C/75 % RH ± 5 %), as may be appreciated from Figure 13.

In order to overcome these drawbacks, the present inventors have researched to develop crystal forms of relacorilant. However, relacorilant is difficult to crystallize. Only an amorphous solid was obtained under different crystallization methods and control of the processing conditions in the preparation process, including: use of single solvents (such as isopropyl ether (IPE) or heptane) or mixture of solvents (such as IPE/heptane or methanol/water) and careful screening of temperature, time, evaporation rate, additives and other factors.

After extensive research, the present inventors have unexpectedly found new solid forms of relacorilant, namely crystalline form I and form II, which have excellent physical and chemical stability, acceptable hygroscopicity, and are suitable for the development of drugs containing relacorilant.

### Definitions

A solid state form, such as a crystal form or amorphous form, may be referred to herein as being characterized by reference to specific data (numerical values or peak positions) as well as to graphical data (e.g. "as depicted in" or "substantially as depicted in" a Figure), determined using spectroscopic and analytical techniques. Such data may include, for example: X-ray powder diffraction or powder X-ray diffraction (XRPD, PXRD, XRD) diffractograms; thermogravimetric analysis (TGA) thermograms. differential scanning calorimetry (DSC) thermograms; or fourier-transform infrared spectroscopy (FTIR) spectra; as well as specific numerical values or peak positions extracted from such graphical representations. As is well-known in the art, the graphical data potentially provides additional technical information to further define the respective solid state form (a so-called "fingerprint") which cannot necessarily be described by reference to numerical values or peak positions alone. In any event, the skilled person will understand that such numerical values or peak positions or graphical representations of data may be subject to small variations, e.g., in peak relative intensities and peak positions due to certain factors such as, but not limited to, variations in instrument response and variations in sample concentration and purity, which are well known to the skilled person. Nonetheless, the skilled person would readily be capable of comparing the graphical data in the Figures herein with graphical data generated for an unknown crystal form and confirm whether the two sets of graphical data are characterizing the same crystal form or two different crystal forms. A crystal form of relacorilant referred to herein as being characterized by graphical data "as depicted in" or "substantially as depicted in" a Figure will thus be understood to include any crystal forms of relacorilant characterized with the graphical data having such small variations, as are well known to the skilled person, in comparison with the Figure.

A solid state form (or polymorph) may be referred to herein as polymorphically pure or as substantially free of any other solid state (or polymorphic) forms. As used herein in this context, the expression "substantially free of any other forms" will be understood to mean that the solid state form contains 20 % or less, 10 % or less, 5 % or less, 2 % or less, or 1 % or less of any other forms of the subject compound as measured, for example, by PXRD. Thus, solid state of relacorilant described herein as substantially free of any other solid state forms would be understood to contain greater than 80 % (w/w), greater than 90 % (w/w), greater than 95 % (w/w), greater than 98 % (w/w), or greater than 99 % (w/w) of the subject solid state form of relacorilant. Accordingly, in some embodiments of the invention, the described solid state forms of relacorilant, crystalline form I and II, may contain from 1 % to 20 % (w/w), from 5 % to 20 % (w/w), or from 5 % to 10 % (w/w) of one or more other solid state forms of the same compound.

A pharmaceutical compound may also exist as a solvate, which is to be understood as any form of the compound which has another molecule (most likely a polar solvent) attached to it via non-covalent bonding. The inventors consider that the crystalline form I of relacorilant of the present invention could actually be an ethyl acetate solvate, including but not being limited to, disolvate, monosolvate or hemisolvate, where the ratio of ethyl acetate molecule to relacorilant molecule is about 2:1, about 1:1 or about 1:2, respectively. The inventors also consider that the crystalline form II of relacorilant of the present invention could actually be a methanol solvate, including but not being limited to, disolvate, monosolvate or hemisolvate, where the ratio of methanol molecule to relacorilant molecule is about 2:1, about 1:1 or about 1:2, respectively.

The amount of solvent employed in a chemical process, e.g., a reaction or crystallization, may be referred to herein as a number of "volumes" or "vol" or "V." For example, a material may be referred to as being suspended (dissolved or otherwise mixed) in 10 volumes (or 10 vol or 10V) of a solvent. In this context, this expression would be understood to mean milliliters of the solvent per gram of the material being suspended (solve dissolved d or otherwise mixed), such that suspending (dissolving or otherwise mixing) 5 grams of a material in 10 volumes of a solvent means that the solvent is used in an amount of 10 milliliters of the solvent per gram of the material that is being suspended (dissolved or otherwise mixed), in this example, 50 mL of the solvent.

By "room temperature" or its abbreviation "rt" is meant herein that the reactions or processes are performed without heating or cooling. Generally, by room temperature may be understood as a temperature between about 15 °C and about 30 °C, or more particularly between about 20 °C and about 25 °C.

As used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. The term "comprises" encompasses the terms "consisting essentially of" and "consisting of". Thus, at each occurrence in the present document, the term "comprising" may be replaced with the term "consisting essentially of" or "consisting of".

As used herein, the term "and/or" includes any and all combinations of one or more of the associated listed items and may be abbreviated as "/". For example, the term "crystalline form I and/or II of relacorilant" includes either or both of forms I and II of relacorilant.

As used herein, the term "approximately" or "about" as applied to one or more values of interest, refers to a value that is similar to a stated reference value. In certain embodiments, the term "approximately" or "about" refers to a value that can vary up to ± 20 %, preferably within ± 10 %, and more preferably within ± 5 % of the stated reference value. When "approximately" or "about" is used before a numerical range, it applies to the upper and lower range end-points.

Indeed, the skilled person knows that numerical values relating to measurements are subject to measurement errors which place limits on their accuracy. Where terms such as "about" or "approximately" are applied to a particular value (e.g., "about 200 °C" or "approximately 200 °C") or to a range (e.g., "about x to approximately y"), the value or range may be interpreted as being as accurate as the method used to measure it. Unless explicitly stated otherwise, the general convention in the scientific and technical literature may be applied so that the last digit of numerical values preferably indicates the precision of measurement. Thus, unless other error margins are given, the maximum margin is preferably ascertained by applying the rounding-off convention to the last decimal place. For instance, a value of 3.5 preferably has an error margin of 3.45 to 3.54 and a range of 2% to 10% preferably covers a range of 1.5% to 10.4%. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 % to about 5 %" should be interpreted to include not only the explicitly recited values of about 1 % to about 5 %, but also include individual values and sub-ranges within the indicated range. Thus, included in this numerical range are individual values such as 2, 3 and 4 and sub-ranges such as from 1-3, from 2-4, and from 3-5, etc. This same principle applies to ranges reciting only one numerical value. It should also be understood that ranges formed by combination of any of the end points of different disclosed ranges and/or particular values therein are included in the present disclosure.

In a first aspect the invention is directed to the crystalline form I of relacorilant. The crystalline forms of relacorilant herein disclosed may be characterized for instance by an X-ray powder diffraction (XRPD), thermogravimetric analysis (TGA), differential scanning calorimetry (DSC) and/or fourier-transform infrared spectroscopy (FTIR).

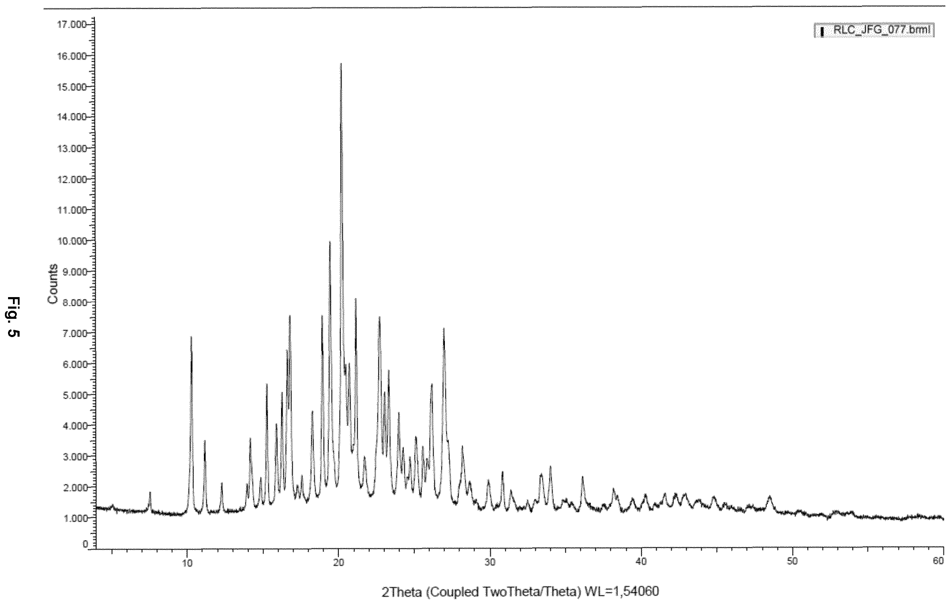

Crystalline form I of relacorilant is primarily characterized by an X-ray powder diffraction (XRPD) pattern having characteristic diffraction peaks at a reflection angle [2θ] of about 20.2±0.2°, 19.5±0.2° and 21.2±0.2°, more precisely of about 20.22±0.2°, 19.47±0.2° and 21.18±0.2°. Particularly, the XRPD pattern further comprises one or more peaks at 2θ degrees of about 19.0±0.2°, 16.8±0.2° and 27.0±0.2°, more precisely of about 18.95±0.2°, 16.82±0.2° and 27.02±0.2°. More particularly, wherein the XRPD pattern further comprises one or more peaks at 2θ degrees of about 10.3±0.2°, 22.8±0.2°, 16.6±0.2° and 20.7±0.2°, more precisely of about 10.32±0.2°, 22.76±0.2°, 16.63±0.2° and 20.72±0.2°. Even more particularly, the XRPD pattern further comprises one or more peaks at 2θ degrees as listed in table 1. Still even more particularly, the XRPD pattern is substantially as depicted in Figure 5. Preferably, the XRPD pattern is measured using an x-ray wavelength of 1.5418Å; more specifically, the 2θ values are obtained using copper radiation (Cu_{Kα1} 1.5418Å). More preferably, XRPD is measured using the equipment and procedure described in the Examples.

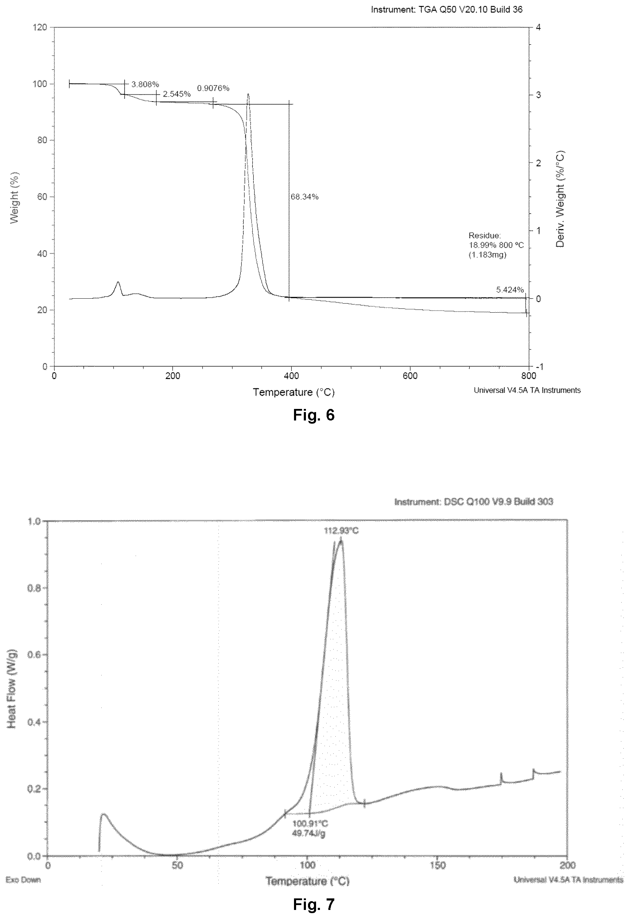

Additionally or alternatively, the crystalline form I of relacorilant is characterized by a thermogravimetric analysis (TGA) thermogram showing a weight loss of about 3.8 % upon heating up to about 120 °C (from room temperature). More particularly, wherein the TGA thermogram further shows a weight loss of about 2.5 % upon heating up to about 170 °C (from room temperature). More particularly, wherein the TGA thermogram shows a total weight loss of about 6.3 % upon heating to about 170 °C, 180 °C, 190 °C or 200 °C (from room temperature). More particularly, the TGA thermogram is substantially as depicted in Figure 6. Preferably, TGA is measured using a heating rate of 10 °C/min. More preferably, TGA is measured using the equipment and procedure described in the Examples.

Additionally or alternatively, the crystalline form I of relacorilant is characterized by a differential scanning calorimetry (DSC) thermogram having an endotherm with an onset of about 100-101 °C, more particularly of about 100.9 °C. More particularly, the DSC thermogram is substantially as depicted in Figure 7. Preferably, DSC is measured using a heating rate of 10 °C/min. More preferably, DSC is measured using the equipment and procedure described in the Examples.

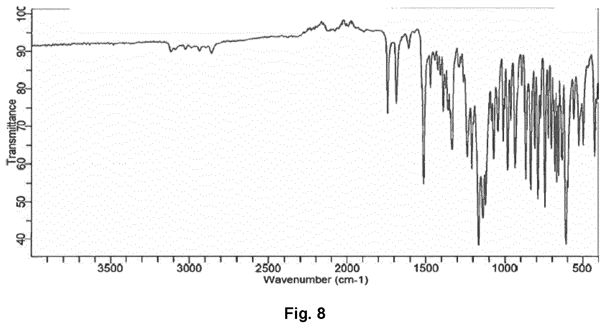

Additionally or alternatively, the crystalline form I of relacorilant is characterized by bands at about 1740, 1686, 1513, 1440, 1420 and 1285 cm⁻¹ in a fourier-transform infrared spectroscopy (FTIR) spectrum. More particularly, the FTIR spectrum is substantially as depicted in Figure 8. More preferably, FTIR is measured using the equipment and procedure described in the Examples.

In a second aspect the invention is directed to the crystalline form II of relacorilant.

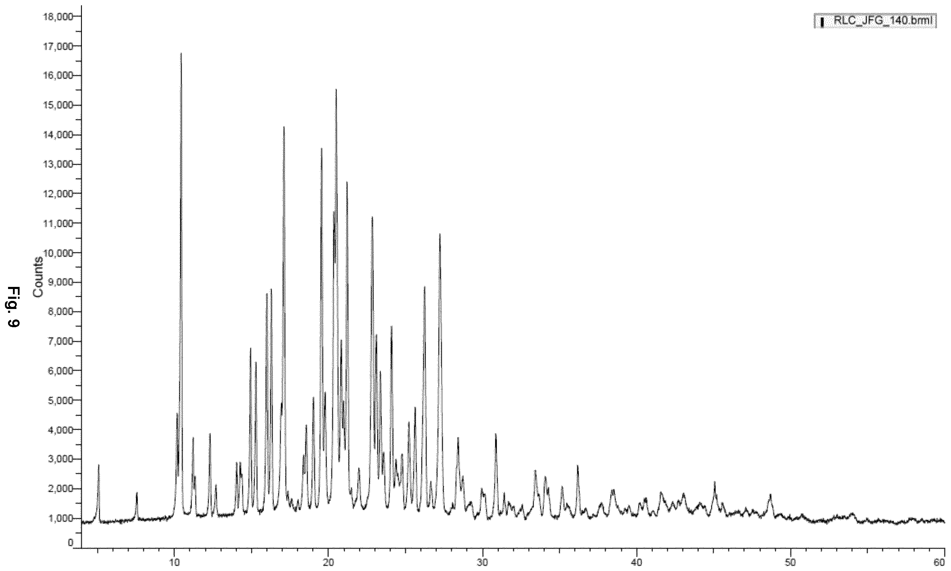

Crystalline form II of relacorilant is primarily characterized by an X-ray powder diffraction (XRPD) pattern having characteristic diffraction peaks at a reflection angle [2θ] of about 10.4±0.2°, 20.5±0.2°, 17.1±0.2° and 19.6±0.2°, more precisely of about 10.45±0.2°, 20.51±0.2°, 17.12±0.2° and 19.56±0.2°. Particularly, the XRPD pattern further comprises one or more peaks at 2θ degrees of about 21.2±0.2°, 20.4±0.2°, 27.2±0.2° and 22.8±0.2°, more precisely of about 21.22±0.2°, 20.36±0.2°, 27.24±0.2° and 22.86±0.2°. More particularly, wherein the XRPD pattern further comprises one or more peaks at 2θ degrees of about 26.2±0.2°, 16.3±0.2°, 16.0±0.2° and 24.1±0.2°, more precisely of about 26.25±0.2°, 16.30±0.2°, 16.01±0.2° and 24.10±0.2°. Even more particularly, the XRPD pattern further comprises one or more peaks at 2θ degrees as listed in table 2. Still even more particularly, the XRPD pattern is substantially as depicted in Figure 9. Preferably, the XRPD pattern is measured using an x-ray wavelength of 1.5418Å; more specifically, the 2θ values are obtained using copper radiation (Cu_{Kα1} 1.5418Å). More preferably, XRPD is measured using the equipment and procedure described in the Examples.

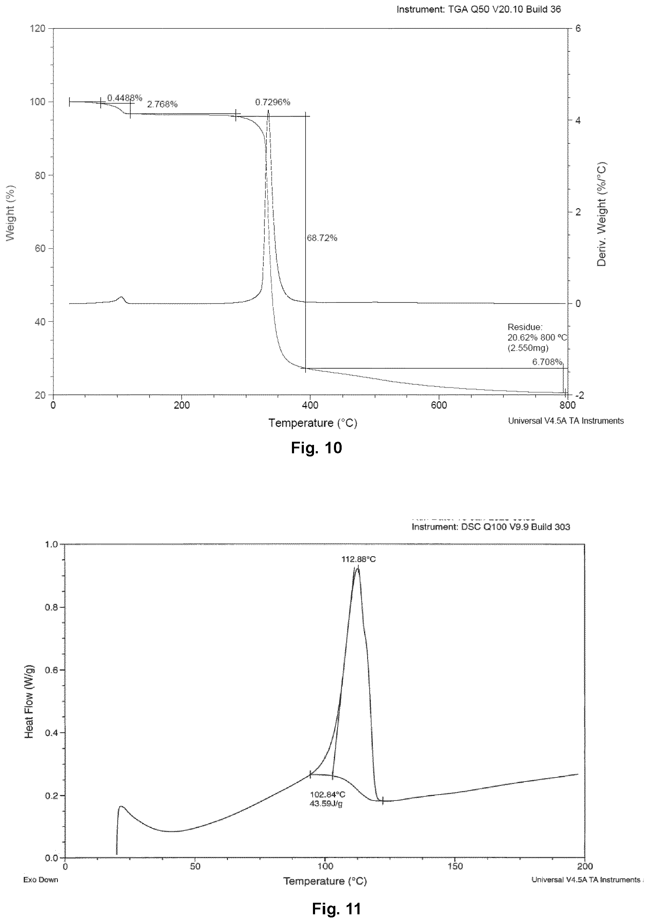

Additionally or alternatively, the crystalline form II of relacorilant is characterized by a thermogravimetric analysis (TGA) thermogram showing a weight loss of about 0.4 % upon heating up to about 75 °C (from room temperature). More particularly, wherein the TGA thermogram further shows a weight loss of about 2.8 % upon heating up to about 120 °C (from room temperature). More particularly, wherein the TGA thermogram shows a total weight loss of about 3.2 % upon heating to about 120 °C, 130 °C, 140 °C or 150 °C (from room temperature). More particularly, the TGA thermogram is substantially as depicted in Figure 10. Preferably, TGA is measured using a heating rate of 10 °C/min. More preferably, TGA is measured using the equipment and procedure described in the Examples.

Additionally or alternatively, the crystalline form II of relacorilant is characterized by a differential scanning calorimetry (DSC) thermogram having an endotherm with an onset of about 102-103 °C, more particularly of about 102.8 °C. More particularly, the DSC thermogram is substantially as depicted in Figure 11. Preferably, DSC is measured using a heating rate of 10 °C/min. More preferably, DSC is measured using the equipment and procedure described in the Examples.

Additionally or alternatively, the crystalline form II of relacorilant is characterized by bands at about 1686, 1515, 1334 and 1164 cm⁻¹ in a fourier-transform infrared spectroscopy (FTIR) spectrum. More particularly, the FTIR spectrum is substantially as depicted in Figure 12. More preferably, FTIR is measured using the equipment and procedure described in the Examples.

A further aspect of the present invention relates a method for preparing the crystalline form I of relacorilant, said method comprising using ethyl acetate as crystallization solvent and optionally adding heptane as anti-solvent until obtaining a suspension. In a particular embodiment, said method comprises dissolving relacorilant in ethyl acetate and optionally adding heptane at room temperature until obtaining a suspension.

In a particular embodiment of the method for preparing the form I, relacorilant is dissolved in about 2 volumes of ethyl acetate. In a particular embodiment, heptane is added in about 2.3 volumes. In a particular embodiment, heptane is added slowly, e.g. dropwise. In a particular embodiment, the suspension is stirred at room temperature for at least about 5, 10, 15, 20, 25 or 30 min. In a particular embodiment, the suspension is filtered to obtain a solid, which is dried for at least about 5, 10, 15, 20, 25, 30, 35, 40, 45, 50, 55 or 60 min.

A further aspect of the present invention relates a method for preparing the crystalline form II of relacorilant, said method comprising using methanol as crystallization solvent. In a particular embodiment, said method comprises mixing relacorilant and methanol to obtain a mixture, heating the mixture until complete solution of the relacorilant and obtaining a suspension by cooling down to room temperature.

In a particular embodiment of the method for preparing the form II, relacorilant is mixed with about 10 volumes of methanol. In a particular embodiment, the mixture is heated, preferably at about 40-50 °C, until complete solution of the relacorilant. In a particular embodiment, after complete solution of the relacorilant, the solution is filtered to remove any insoluble impurities. In particular embodiment, after filtering the solution, methanol is added for washing, and then optionally partially distilled. In a particular embodiment, the suspension is filtered to obtain a solid, which is dried.

A further aspect of the present invention relates to a crystalline form of relacorilant obtainable by any of the above methods.

A further aspect of the present invention relates to a pharmaceutical composition or a medicament comprising the crystalline form I and/or II of relacorilant and a pharmaceutically acceptable excipient.

Examples of pharmaceutical compositions include any solid (tablets, pills, capsules, granules, etc.), semi-solid (creams, ointments, etc.) or liquid (solutions, suspensions or emulsions) composition.

The compositions can for example be administered orally, topically, dermally, nasally, intravenously, intramuscularly, intraperitoneally, intracerobrospinally, intracranially, intraspinally, subcutaneously, intraarticularly, intrasynovialy, or intrathecaly. Other forms of administration are not excluded. According to a particular embodiment, said composition is for oral administration. In a more particular embodiment, the crystalline form I and/or II of relacorilant is formulated as a capsule for oral administration.

The respective composition or medicament may - depending on its route of administration - also contain one or more excipients known to those skilled in the art. The composition or medicament according to the present invention may be produced according to standard procedures known to those skilled in the art.

The term "excipient" refers to components of a drug compound other than the active ingredient (definition obtained from the European Medicines Agency- EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong in vivo drug actions, decrease drug metabolism, and reduce drug toxicity. Carriers are also used in designs to increase the effectiveness of drug delivery to the target sites of pharmacological actions (U.S. National Library of Medicine. National Institutes of Health). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, ^{©} 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

This invention also refers to the crystalline form I and/or II of relacorilant for use in medicine, particularly for the treatment and/or prophylaxis of Cushing's syndrome, solid tumors or alcoholism.

Likewise, this invention also refers to the use of the crystalline form I and/or II of relacorilant in the manufacture of a medicament, particularly a medicament for the treatment and/or prophylaxis of Cushing's syndrome, solid tumors or alcoholism.

Likewise, this invention also refers to a method for the treatment of a patient, notably a human, suffering or likely to suffer Cushing's syndrome, solid tumors or alcoholism, which comprises administering to the patient in need of such a treatment or prophylaxis an effective amount of the crystalline form I and/or II of relacorilant.

Examples of solid tumors which may be treated and/or prevented with the crystalline form I and/or II of relacorilant include, but are not limited to, ovarian, adrenal, prostate, pancreatic, and triple-negative breast.

As used herein, the terms "treat", "treating" and "treatment" include in general the eradication, removal, reversion, alleviation, modification, or control of a disease after its onset.

As used herein, the terms "prevention", "preventing", "preventive", "prevent" and "prophylaxis" refer to the capacity of a given substance, composition or medicament to avoid, minimize or difficult the onset or development of a disease before its onset.

By an "effective" amount or a "therapeutically effective amount" of a drug or pharmacologically active agent is meant a nontoxic but sufficient amount of the drug or agent to provide the desired effect. In the therapy of the present invention, an "effective amount" of the crystalline form I and/or II of relacorilant is the amount of that compound that is effective to provide the desired effect. The amount that is "effective" will vary from subject to subject, depending on the age and general condition of the individual, the particular active agent or agents, and the like. Thus, it is not always possible to specify an exact "effective amount". However, an appropriate "effective" amount in any individual case may be determined by one of ordinary skill in the art using routine experimentation. In a particular embodiment, the crystalline forms I and/or II of relacorilant is administered as a total dose of 100-400 mg per day.

The present invention also encompasses the crystalline form I and/or II of relacorilant with other drugs (e.g. an anticancer drug such as nab-paclitaxel). A combination of the crystalline form I and/or II of relacorilant and at least another drug may be formulated for its simultaneous, separate or sequential administration. This has the implication that the combination of the two compounds (i.e. crystalline form(s) and the other drug) may be administered:
- as a combination that is being part of the same medicament formulation, the two compounds being then administered always simultaneously.
- as a combination of two units, each with one of the substances giving rise to the possibility of simultaneous, sequential or separate administration.

In a particular embodiment, the crystalline form I and/or II of relacorilant is independently administered from the other drug (i.e in two units) but at the same time. In another particular embodiment, the crystalline form I and/or II of relacorilant is administered first, and then the other drug is separately or sequentially administered. In yet another particular embodiment, the other drug is administered first, and then the crystalline form I and/or II of relacorilant is administered, separately or sequentially, as defined.

The following examples are merely illustrative of certain embodiments of the invention and cannot be considered as restricting it in any way.

### EXAMPLES

### The following equipment and procedures were used in the characterization of solid forms of relacorilant

### a) X-ray powder diffraction analysis (XRPD)

Approximately 20 mg of product samples were prepared in standard sample holders using two foils of polyacetate.

X-Ray Powder Difraction (XRPD) patterns of the samples were obtained using a Bruker D8 Advance X-Ray diffractometer with DaVinci Geometry, with motor support between Gobel mirror for parallel-beam geometry and a motorized divergence slit for Bragg-Brentano geometry equipped with goniometer radius 420 mm and LynxEye XE detector under the following conditions: using Cu-Kα source with a wavelength of 1.5418 Å without monochromator in 4-60° 2 Theta range (step size 0.0170°; time/step 1 s; Soller slit 2.5°, antiscatter slit 9 mm, divergence slit 6 mm; current 40 mA and voltage 40 KV).

### b) Thermogravimetric analysis (TGA)

TGA analyses were carried out on a TA Instruments TGA Q50 953501-901. Approximately 2.0 mg of samples were placed in a tared platinum or aluminum pan, automatically weighed, and inserted into the TGA furnace. The samples were heated at 10 °C/min to a final temperature of 800° C. The purge gas is nitrogen for balance at 40 mL/min and the sample at 60 mL/min, respectively.

### c) Differential Scanning Calorimetry analysis (DSC)

Differential Scanning Calorimetry (DSC) experiments were performed using a Differential Scanning Calorimeter: Perkin-Elmer DSC7, connected to a computer with a Perkin-Elmer TAC 7/DX interface. The sample (about 1-10 mg) was weighed in an aluminum pan. The instrument was purged with nitrogen gas at 50 mL/min. Data were collected between room temperature and 300 °C at a heating rate of 10 °C/min.

### d) Fourier-Transform Infrared spectroscopy (FTIR)

Infrared (IR) spectra were recorded directly by means of an Agilent Cary 630 FTIR model Spectrum BX Fourier transform spectrophotometer.

### Example 1. Amorphous relacorilant (comparative)

Relacorilant in amorphous form was obtained using one single solvent (e.g. isopropyl ether (IPE) or heptane) or mixture of solvents (e.g. isopropyl ether/heptane or methanol/water).

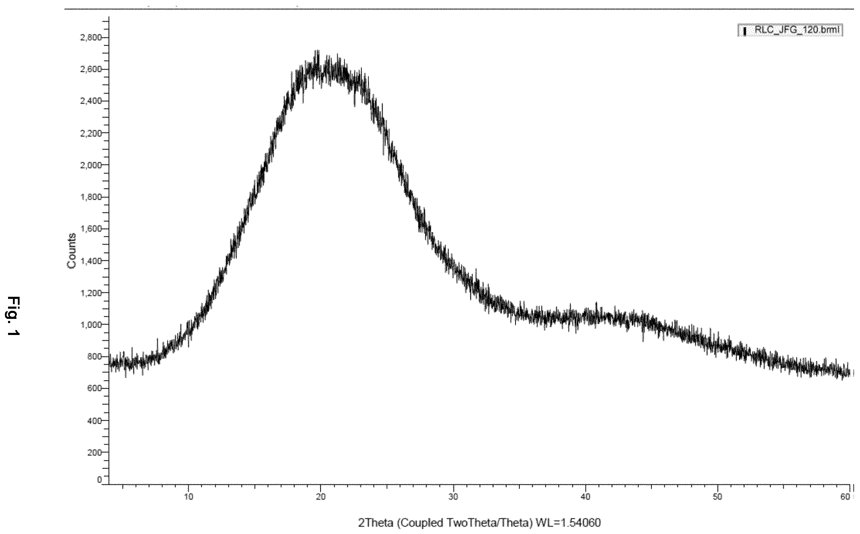

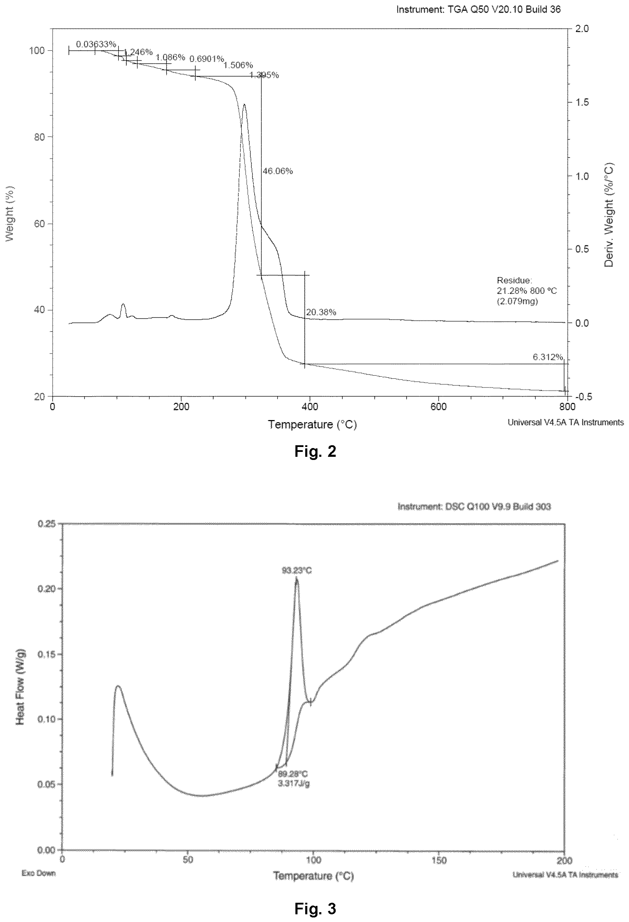

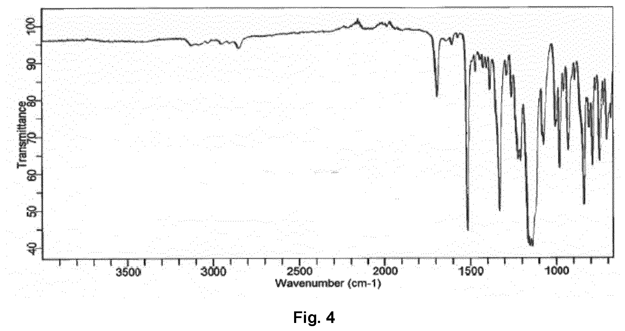

By way of illustration, the preparation of amorphous relacorilant from methanol/water is disclosed below:
800.0 mg of solid relacorilant were weighed and placed in a glass vial and then 14.4 mL of MeOH were added. The mixture was heated at 40°C until complete dissolution. The solution was added over 20 mL of water at 20/25°C slowly. It was stirred for 1 hour at 20/25°C. The solid obtained was filtered, washed with water and dried under vacuum at 50°C, and analyzed by XRPD, TGA and FTIR (see Figures 1, 2, 3 and 4).

Relacorilant in amorphous form was subjected to stability studies at accelerated storage conditions of 40 °C ± 2 °C/75 % RH ± 5 % and some degradation was detected at only 1 month by XRPD (see Figure 13).

### Example 2. Crystalline form I of relacorilant

300.0 mg of solid relacorilant were weighed and placed in a glass vial and then 0.6 mL of EtOAc were added. The mixture was stirred until complete solution. 0.7 mL of heptane were added slowly at room temperature. A suspension was obtained and was stirred at room temperature for 30 minutes. The solid obtained was filtered, separated, dried for 1 hour to afford 110 mg of relacorilant crystalline form I, which was analyzed by XRPD, TGA, DSC and IR (see Figures 5, 6, 7 and 8).The following table shows the peak list of the XRPD of relacorilant crystalline form I:

**Table 1**

| **Index** | **Angle** | **D Value** | **Intensity** | **Rel. Intensity** |
|---|---|---|---|---|
| 1 | 4.991 ° | 17.69209 Å | 1340.13 Counts | 0.6 % |
| 2 | 7.552 ° | 11.69642 Å | 1646.13 Counts | 4.0 % |
| 3 | 10.315 ° | 8.56881 Å | 5058.39 Counts | 33.6 % |
| 4 | 11.188 ° | 7.90232 Å | 2779.65 Counts | 13.9 % |
| 5 | 12.296 ° | 7.19243 Å | 1842.68 Counts | 5.6 % |
| 6 | 13.945 ° | 6.34543 Å | 1872.01 Counts | 5.0 % |
| 7 | 14.177 ° | 6.24213 Å | 2877.43 Counts | 13.3% |
| 8 | 14.853 ° | 5.95948 Å | 2009.22 Counts | 5.3 % |
| 9 | 15.274 ° | 5.79640 Å | 4192.74 Counts | 23.7 % |
| 10 | 15.904 ° | 5.56810 Å | 3288.28 Counts | 15.5 % |
| 11 | 16.277 ° | 5.44111 Å | 3990.48 Counts | 21.3 % |
| 12 | 16.628 ° | 5.32705 Å | 5009.77 Counts | 29.9 % |
| 13 | 16.817 ° | 5.26771 Å | 5685.69 Counts | 35.6 % |
| 14 | 17.260 ° | 5.13365 Å | 1946.87 Counts | 3.5 % |
| 15 | 17.574 ° | 5.04248 Å | 2156.65 Counts | 5.2 % |
| 16 | 18.295 ° | 4.84547 Å | 3503.6 Counts | 16.4 % |
| 17 | 18.953 ° | 4.67864 Å | 6000.49 Counts | 37.3 % |
| 18 | 19.468 ° | 4.55603 Å | 7611.42 Counts | 50.8 % |
| 19 | 20.221 ° | 4.38790 Å | 13390.6 Counts | 100.0 % |
| 20 | 20.486 ° | 4.33175 Å | 4553.75 Counts | 24.3 % |
| 21 | 20.724 ° | 4.28261 Å | 4795.64 Counts | 26.4 % |
| 22 | 21.183 ° | 4.19079 Å | 6427.3 Counts | 40.3 % |
| 23 | 21.731 ° | 4.08636 Å | 2603.45 Counts | 7.6 % |
| 24 | 22.757 ° | 3.90440 Å | 5506.5 Counts | 32.3 % |
| 25 | 23.069 ° | 3.85237 Å | 4195.39 Counts | 21.1 % |
| 26 | 23.338 ° | 3.80845 Å | 4733.55 Counts | 25.7 % |
| 27 | 24.005 ° | 3.70418 Å | 3738.82 Counts | 17.3 % |
| 28 | 24.293 ° | 3.66091 Å | 2821 Counts | 9.5 % |
| 29 | 24.721 ° | 3.59852 Å | 2601.86 Counts | 7.8 % |
| 30 | 25.126 ° | 3.54137 Å | 3130.18 Counts | 12.5 % |
| 31 | 25.600 ° | 3.47688 Å | 2886.02 Counts | 10.7 % |
| 32 | 25.871 ° | 3.44111 Å | 2517.93 Counts | 7.7 % |
| 33 | 26.174 ° | 3.40192 Å | 4018.72 Counts | 20.7 % |
| 34 | 27.015 ° | 3.29788 Å | 5483.87 Counts | 33.9 % |
| 35 | 27.272 ° | 3.26743 Å | 2852.25 Counts | 11.7 % |
| 36 | 28.222 ° | 3.15954 Å | 2820.13 Counts | 12.3 % |
| 37 | 28.678 ° | 3.11030 Å | 1923.63 Counts | 5.0 % |
| 38 | 29.086 ° | 3.06767 Å | 1480.79 Counts | 1.7 % |
| 39 | 29.888 ° | 2.98707 Å | 1941.8 Counts | 5.9 % |
| 40 | 30.837 ° | 2.89733 Å | 2195.13 Counts | 8.1 % |
| 41 | 31.380 ° | 2.84838 Å | 1712.57 Counts | 4.0 % |
| 42 | 32.490 ° | 2.75356 Å | 1469.42 Counts | 2.0 % |
| 43 | 33.004° | 2.71183 Å | 1470.63 Counts | 2.0 % |
| 44 | 33.395 ° | 2.68102 Å | 1969.5 Counts | 6.2 % |
| 45 | 34.005 ° | 2.63429 Å | 2300.39 Counts | 9.0 % |

### Example 3. Crystalline form II of relacorilant

200.0 mg of solid relacorilant were weighed and placed in a glass vial and then 2.0 mL of MeOH were added. The mixture was heated at 40/50°C and stirred until complete solution of the relacorilant. The solution was filtered to remove any insoluble impurities and washed with MeOH 0.5 mL. The filtrate was stirred until room temperature and stirred overnight and a suspension was obtained. The solid obtained was filtered and dried to afford relacorilant crystalline form II, which was analyzed by XRPD, TGA, DSC and IR (see Figures 9, 10, 11 and 12). The following table shows the peak list of the XRPD of relacorilant crystalline form II:

**Table 2**

| **Index** | **Angle** | **D Value** | **Intensity** | **Rel. Intensity** |
|---|---|---|---|---|
| 1 | 5.089 ° | 17,35176 | 2097.56 Counts | 11.3% |
| 2 | 7.573 ° | 11,6649 | 1547.94 Counts | 5.7% |
| 3 | 10.185 ° | 8,67825 | 3416.55 Counts | 21.9% |
| 4 | 10.448 ° | 8,46022 | 11927.8 Counts | 100.0% |
| 5 | 11.219 ° | 7,88072 | 2861.73 Counts | 16.6% |
| 6 | 11.348 ° | 7,79122 | 1982.51 Counts | 8.5% |
| 7 | 12.313 ° | 7,18247 | 3074.88 Counts | 18.3% |
| 8 | 12.699 ° | 6,96512 | 1803.2 Counts | 6.5% |
| 9 | 14.047 ° | 6,29954 | 2397.85 Counts | 11.3% |
| 10 | 14.279 ° | 6,19774 | 2383.56 Counts | 11.0% |
| 11 | 14.949 ° | 5,92137 | 5098.38 Counts | 35.5% |
| 12 | 15.298 ° | 5,78736 | 4917.41 Counts | 33.7% |
| 13 | 16.006 ° | 5,53284 | 6465.84 Counts | 47.6% |
| 14 | 16.300 ° | 5,4335 | 6701.69 Counts | 49.7% |
| 15 | 16.940 ° | 5,22986 | 3735.21 Counts | 22.3% |
| 16 | 17.117 ° | 5,17597 | 10434.5 Counts | 83.9% |
| 17 | 17.374 ° | 5,10009 | 1749.47 Counts | 4.1% |
| 18 | 17.606 ° | 5,03346 | 1554.41 Counts | 2.3% |
| 19 | 18.033 ° | 4,91516 | 1535.04 Counts | 2.0% |
| 20 | 18.396 ° | 4,81906 | 2566.71 Counts | 11.3% |
| 21 | 18.562 ° | 4,77615 | 3411.73 Counts | 19.0% |
| 22 | 19.030 ° | 4,65989 | 4091.9 Counts | 25.0% |
| 23 | 19.559 ° | 4,535 | 10334.3 Counts | 82.2% |
| 24 | 19.786 ° | 4,48351 | 4085.61 Counts | 24.8% |
| 25 | 20.360 ° | 4,35832 | 8699.55 Counts | 67.1% |
| 26 | 20.509 ° | 4,32692 | 11983.9 Counts | 97.3% |
| 27 | 20.829 ° | 4,26136 | 5585.85 Counts | 38.6% |
| 28 | 20.984 ° | 4,23006 | 4150.28 Counts | 25.4% |
| 29 | 21.218 ° | 4,18396 | 9851.79 Counts | 77.8% |
| 30 | 21.471 ° | 4,1352 | 1766.45 Counts | 3.6% |
| 31 | 21.737 ° | 4,0853 | 1564.23 Counts | 1.8% |
| 32 | 21.978 ° | 4,04105 | 2335.55 Counts | 9.0% |
| 33 | 22.855 ° | 3,88784 | 8075.24 Counts | 61.7% |
| 34 | 23.116 ° | 3,8446 | 5577.01 Counts | 38.7% |
| 35 | 23.376 ° | 3,80237 | 4922.88 Counts | 32.7% |
| 36 | 23.588 ° | 3,76868 | 2699.16 Counts | 12.3% |
| 37 | 24.100 ° | 3,68976 | 6134.32 Counts | 43.8% |
| 38 | 24.387 ° | 3,647 | 2552.69 Counts | 11.0% |
| 39 | 24.766 ° | 3,59209 | 2807.2 Counts | 13.4% |
| 40 | 25.226 ° | 3,52753 | 3609.97 Counts | 21.0% |
| 41 | 25.630 ° | 3,47285 | 3985.1 Counts | 24.6% |
| 42 | 26.248 ° | 3,39254 | 6727.5 Counts | 50.1% |
| 43 | 26.649 ° | 3,34235 | 2039.64 Counts | 7.2% |
| 44 | 27.244 ° | 3,27067 | 7936.77 Counts | 61.8% |
| 45 | 28.062 ° | 3,17715 | 1443.48 Counts | 2.7% |

### Example 4. Crystalline form II of relacorilant

200.0 mg of solid relacorilant were weighed and placed in a glass vial and then 2.0 mL of MeOH were added. The mixture was heated at 40/50°C and stirred until complete solution of the relacorilant. The solution was filtered to remove any insoluble impurities. MeOH (4 times 2.0 mL) were added and the solvent was distilled under vacuum at 40°C each time to a volume of 2.0 mL. It was stirred at room temperature and a suspension was obtained. The solid obtained was filtered and dried to afford 110 mg of relacorilant crystalline form II.

## Claims

1. A crystalline form of relacorilant, designated herein as crystalline form I, **characterized by** an X-ray powder diffraction (XRPD) pattern comprising diffraction peaks at 2θ degrees of about 20.2±0.2°, 19.5±0.2° and 21.2±0.2°.

2. The crystalline form according to claim 1, wherein the XRPD pattern further comprises one or more peaks at 2θ degrees of about 19.0±0.2°, 16.8±0.2° and 27.0±0.2°; and/or wherein the XRPD pattern further comprises one or more peaks at 2θ degrees of about 10.3±0.2°, 22.8±0.2°, 16.6±0.2° and 20.7±0.2°; and/or wherein the XRPD pattern further comprises one or more peaks at 2θ degrees as listed in table 1; and/or wherein the XRPD pattern is substantially as depicted in Figure 5.

3. The crystalline form according to any one of claims 1 to 2, wherein the crystalline form I of relacorilant is **characterized by** a thermogravimetric analysis (TGA) showing a weight loss of about 3.8 % upon heating up to about 120 °C; more particularly, wherein the TGA thermogram further shows a weight loss of about 2.5 % upon heating up to about 170 °C; and/or wherein the crystalline form I of relacorilant has a TGA thermogram substantially as depicted in Figure 6.

4. The crystalline form according to any one of claims 1 to 3, wherein the crystalline form I of relacorilant is **characterized by** a differential scanning calorimetry (DSC) thermogram having an endotherm with an onset of about 100-101 °C; and/or wherein the crystalline form I of relacorilant has a DSC thermogram substantially as depicted in Figure 7

5. The crystalline form according to any one of claims 1 to 4, wherein the crystalline form I of relacorilant is **characterized by** bands at about 1740, 1686, 1513, 1440, 1420 and 1285 cm⁻¹ in a fourier-transform infrared spectroscopy (FTIR) spectrum; and/or wherein the crystalline form I of relacorilant has a FTIR spectrum substantially as depicted in Figure 8.

6. A crystalline form of relacorilant, designated herein as crystalline form II, **characterized by** an X-ray powder diffraction (XRPD) pattern comprising diffraction peaks at 2θ degrees of about 10.4±0.2°, 20.5±0.2°, 17.1±0.2° and 19.6±0.2°.

7. The crystalline form according to claim 6, wherein the XRPD pattern further comprises one or more peaks at 2θ degrees of about 26.2±0.2°, 16.3±0.2°, 16.0±0.2° and 24.1±0.2°; and/or wherein the XRPD pattern further comprises one or more peaks at 2θ degrees as listed in table 2; and/or wherein the XRPD pattern is substantially as depicted in Figure 9.

8. The crystalline form according to any one of claims 6 to 7, wherein the crystalline form II of relacorilant is **characterized by** a thermogravimetric analysis (TGA) showing a weight loss of about 0.4 % upon heating up to about 75 °C; more particularly, wherein the TGA thermogram further shows a weight loss of about 2.8 % upon heating up to about 120 °C; and/or wherein the crystalline form II of relacorilant has a TGA thermogram substantially as depicted in Figure 10.

9. The crystalline form according to any one of claims 6 to 8, wherein the crystalline form II of relacorilant is **characterized by** a differential scanning calorimetry (DSC) thermogram having an endotherm with an onset of about 102-103 °C; and/or wherein the crystalline form II of relacorilant has a DSC thermogram substantially as depicted in Figure 11.

10. The crystalline form according to any one of claims 6 to 9, wherein the crystalline form II of relacorilant is **characterized by** bands at about 1686, 1515, 1334 and 1164 cm⁻¹ in a fourier-transform infrared spectroscopy (FTIR) spectrum; and/or wherein the crystalline form II of relacorilant has a FTIR spectrum substantially as depicted in Figure 12.

11. A method for preparing the crystalline form I of relacorilant as defined in any one of claims 1 to 5, said method using ethyl acetate as crystallization solvent and optionally adding heptane as anti-solvent until obtaining a suspension; preferably wherein relacorilant is dissolved in about 2 volumes of ethyl acetate; and/or wherein heptane is added in about 2.3 volumes; and/or wherein heptane is added dropwise; and/or wherein the suspension was stirred at room temperature for at least about 5, 10, 15, 20, 25 or 30 min; and/or wherein the suspension is filtered to obtain a solid, which is dried for at least about 5, 10, 15, 20, 25, 30, 35, 40, 45, 50, 55 or 60 min.

12. A method for preparing the crystalline form II of relacorilant as defined in any one of claims 6 to 10, said method using methanol as crystallization solvent; preferably wherein said method comprises mixing relacorilant and methanol to obtain a mixture, heating the mixture until complete solution of the relacorilant and obtaining a suspension by cooling down to room temperature.

13. A crystalline form of relacorilant, designated herein as crystalline form I, obtainable by the method according to claim 11 or a crystalline form of relacorilant, designated herein as crystalline form II, obtainable by the method according to claim 12.

14. A pharmaceutical composition comprising the crystalline form I of relacorilant as defined in any one of claims 1 to 5 or 13 and/or the crystalline form II of relacorilant as defined in any one of claims 6 to 10 or 13 and a pharmaceutically acceptable excipient.

15. The crystalline form I of relacorilant as defined in any one of claims 1 to 5 or 13 and/or the crystalline form II of relacorilant as defined in any one of claims 6 to 10 or 13 for use in medicine, more particularly for use in the treatment and/or prevention of Cushing's syndrome, solid tumors or alcoholism.
